# EUROPEAN PATENT APPLICATION

(11) **EP 4 834 342 A1**
(43) Date of publication of application: **04.11.2026**
(21) Application number: 24909255.2
(22) Date of filing: 27.12.2024
(51) Int. Cl.: A61K 31/42, A61K 9/20, A61P 33/14

(54) **COMPOSITION FOR DOMESTIC ANIMALS, PRODUCTION PROCESS AND USE OF COMPOSITION**

(30) Priority: 29.12.2023 BR 102023027877
(71) Applicant: Ouro Fino Saúde Animal Ltda., 14140000 Cravinhos (BR)
(72) Inventor: MULLER, Vinicius, 14140-000 Cravinhos (BR); GATTO, Igor Renan Honorato, 14140-000 Cravinhos (BR); CRUZ, Breno Cayeiro, 14140-000 Cravinhos (BR); TAGAVA, Patrícia Chiba, 14140-000 Cravinhos (BR); ALVIM JUNIOR, Joel, 14140-000 Cravinhos (BR); AKIMURA, Tatiana Okura, 14140-000 Cravinhos (BR); GOMES, Jeferson, 14140-000 Cravinhos (BR)
(74) Representative: Clarke, Modet y Cía., S.L.
(86) International application number: PCT/BR2024/050603
(87) International publication number: WO 2025/137755

(57) **Abstract**

The invention provides oral solid compositions comprising poorly water-soluble active ingredients at high concentrations with high bioavailability in a reduced size solid composition. These compositions are obtained in the present invention through a sustainable manufacture process, which eliminates the need for solvents and heating. The invention also discusses the use of such compositions for the production of veterinary drugs such as insecticides and/or acaricides.

## Description

### Field of the invention

The present invention is in the field of veterinary medicine, more specifically, in the field of anhydrous veterinary compositions for domestic animals. The invention overcomes the challenge of delivering oral solid compositions comprising poorly water-soluble active ingredients at high concentrations. Surprisingly, the present composition also provides high bioavailability in a reduced size solid composition. These compositions are obtained in the present invention through a sustainable manufacture process, which eliminates the need for solvents and heating. The invention also discusses the use of such compositions for the production of veterinary medicines such as insecticides and/or acaricides.

### Background of the Invention

Animals require special care regarding parasitic diseases that can infect them. Domestic animals, in particular, which live in close proximity to humans, need ways to prevent and treat parasitic diseases and their associated illnesses, in order to avoid harming the animals' health, as well as preventing the contamination of humans.

However, the development of oral compositions containing high concentrations of active ingredients presents pharmaceutical challenges that must be overcome. Some of these challenges include developing a formulation with an appropriate size for easy ingestion by the animals, especially when that composition has a high concentration of active ingredient. Other challenges relate to the bioavailability of the oral composition, which must be adequate so that the active ingredient is released and kept in the body long enough for its effect to be perceived. Another challenge concerns the process for manufacturing such compositions, since ideally it should be efficient, sustainable, and capable of maintaining the physicochemical properties of the molecules until they reach their destination.

In particular, in the present invention, we have observed that some processes for manufacturing oral compositions with moisture can lead to the hydrolysis of active molecules that are poorly water soluble over a wide pH range. Therefore, certain processes and parameters must be adopted to achieve a suitable active product.

Furthermore, there is a need for such manufacture processes to be sustainable, helping the environment and the nature.

Thus, the present invention comprises oral solid compositions containing poorly water soluble compounds, more specifically, isoxazoline active ingredients, in high concentrations. These compositions are shown to present high bioavailability of the compound in the domestic animals' organism and are found in reduced sizes considering the high concentrations of compounds present in said compositions.

Moreover, there is also provided a manufacture process that eliminates the use of organic solvents, which can be carcinogenic or toxic, thus preventing their dispersion into the environment.

We have reviewed some state-of-the-art documents that may potentially be related to the present invention, and these are exemplified below.

Document BR 11 2014 024832 0 comprises a soft chewable tablet with specific organic solvents (such as 2-pyrrolidone and DMAC) containing low amounts of isoxazolines. Document BR 11 2021 004218 0 describes a chewable tablet with a combination of low amounts of isoxazolines and macrocyclic lactones, in addition to other active ingredients such as the anthelmintic pyrantel palmoate, and having a hardness of at least 30 N. Finally, document BR 11 2020 008853 6 comprises isoxazolines with a specific particle size distribution (D50 from 25 to 250 microns) in an injectable formulation.

Thus, none of the prior art documents were able to anticipate the aspects of the present invention, either individually or in combination, among which we highlight: - compositions having a high concentration of active ingredients that are poorly soluble in water (low flowability and/or compressibility), which make it difficult to manufacture a solid form composition; - compositions having an appropriate relationship between high concentration of active ingredient and the composition size, in addition to high bioavailability; - a sustainable manufacture process, being free of organic solvents and heating; - the use of said composition as an insecticide or acaricide. Thus, the present invention provides a unique and inventive composition, as well as its manufacture process and potential uses.

### Summary of the invention

The present invention comprises highly bioavailable solid veterinary compositions containing a high concentration of poorly water-soluble active ingredients.

More specifically, the present invention comprises a composition for domestic animals comprising:
i) 30 to 70% of at least one poorly water-soluble active ingredient;
ii) 30 to 70% of at least one excipient.

In a preferred embodiment, the aforementioned solid composition in oral form has a diameter of less than 10 mm for compositions up to 200 mg, less than 15 mm for compositions from 200 to 800 mg, and less than 30 mm for compositions from 800 mg to 1200 mg.

Also, the size to hardness ratio of the composition is 0.5-1.5.

In a preferred embodiment, the composition of the present invention is an anhydrous solid veterinary composition.

In a preferred embodiment, the composition comprises an active ingredient concentration of about 50% in the composition.

In a preferred embodiment, the excipients comprise at least one of the following: binder, glidant, disintegrant, surfactant, palatability enhancer, lubricant, and/or diluent.

In a preferred embodiment, the excipient of said composition comprises at least one of the following: - 1 to 10% w/w binder; - 0.1 to 5% w/w surfactant, glidant, lubricant or disintegrant; - 1 to 20% w/w palatability enhancer; - 5% to 50% w/w diluent.

The present invention further comprises a sustainable manufacture process for obtaining solid veterinary compositions comprising active ingredients that are poorly water-soluble.

The manufacture process of the present invention eliminates the need for solvents and heating means.

In a preferred embodiment, the manufacture process comprises the following steps:
a) mixing part of the diluent with the active ingredient;
b) mixing part of the diluent with part of the glidant;
c) mixing a) with part of the diluent and homogenizing;
d) mixing b), the surfactant, disintegrant, binder, c) and homogenizing;
e) compacting the mixture of d);
f) obtaining granules and transferring the compacted mixture to the mixer of e);
g) adding and mixing with the remaining disintegrant, glidant, lubricant, total palatability enhancer, and diluent.

In a preferred embodiment, the granules are obtained using the dry granulation technique.

The aforementioned manufacture process exhibits high yield, enabling the rapid and highly efficient production of solid oral compositions.

The present invention further comprises the use of said composition for the treatment of veterinary diseases.

### Description of the Invention and Examples

The above examples should not be seen as limiting, but rather as proof of concept of the present invention.

### Oral solid compositions

The oral solid compositions of the present invention comprise a high concentration of poorly water-soluble active ingredients. The poorly water-soluble active ingredients selected for the invention comprise active ingredients having water solubility of less than 1 mg/mL at 25° C. More specifically, they comprise isoxazoline compounds. Even more preferably, they comprise the compounds fluralaner, sarolaner, afoxolaner, and lotilaner.

In another embodiment, the high concentration of active ingredients comprises values between 30 and 70% of actives in the composition, more specifically 40 to 60%, preferably 45 to 55%, and even more preferably 50%.

Determining the high bioavailability of said composition comprises measuring the areas under the curve (AUC) and Cmax, and comparing compositions containing the elements of the present invention with compositions lacking at least one of these elements, in order to demonstrate that the composition of the present invention can enhance the bioavailability of the active ingredient in domestic animals.

The oral solid compositions of the present invention have a reduced size in mm relative to their concentration of active ingredients. For example, a formulation weighing 90 mg comprising 50% by weight of a poorly water-soluble active ingredient (45 mg) has a size smaller than 10 mm, preferably 6 mm (round). In general, the solid oral composition of the present invention comprises a diameter of less than 10 mm for compositions up to 200 mg, less than 15 mm for compositions from 200 to 800 mg, and less than 30 mm for compositions from 800 mg to 1200 mg.

The composition size is associated with the appropriate dosage of the poorly water-soluble active ingredient according to the weight and/or size of the animal, with presentations ranging from 10 to 2000 mg, or more specifically from 50 to 1500 mg, and even more preferably from 90, 200, 400, 800 and 1120 mg. Each component has a weight proportional to its size, according to the aforementioned relationships. In a preferred embodiment, the average weight of the composition ranges from 5 to 3000 mg, even more preferably from 25 to 2250 mg, even more preferably from 80 to 90 mg, from 180 to 220 mg, from 370 to 430 mg, from 750 to 850 mg and from 1000 to 1200 mg.

Preferably, the present invention comprises an optimal relationship between the active ingredient concentration in the composition (weight in mg) and the tablet size (diameter in mm) of 5 to 15 mm for tablets containing up to 150 mg of active ingredient, 10 to 30 mm for tablets containing from 50 mg to 250 mg of active ingredient, and 25 to 50 mm for tablets containing more than 200 mg of active ingredient.

Solid dosage forms for oral administration include capsules, tablets, pills, powders, and granules, preferably tablets having a hardness of about 1, 2, 3, 4, 5, 6, 7, 8, 9, 10, 11, 12, 13, 14, 15, 16, 17, 18, 19, 20 Kgf, and the useful ranges can be selected from any of these values. The hardness of the solid oral composition of the present invention can also be seen in the relationship between the average hardness of the tablet (Kgf) and the size of the tablet (mm), where a surprising ratio of 0.5 to 1.5 was observed, which characterizes the solid oral composition of the present invention from a pharmacotechnical standpoint.

In such solid dosage forms, the active compound is mixed with:
a) from 5 to 50% or in quantities sufficient to complete the expected weight of the composition (q.s.p.) of at least one pharmaceutically acceptable inert excipient or carrier or diluent, such as sodium citrate or dicalcium phosphate starches, lactose, sucrose, glucose, mannitol, silicic acid, talc and silicified microcrystalline cellulose;
b) from 1 to 10%, even more preferably 1%, 2%, 3%, 4%, 5%, 6%, 7%, 8%, 9% or 10% of binders such as, for example, cellulose derivatives, such as carboxymethylcellulose, methylcellulose, hydroxypropylcellulose, hydroxypropylmethylcellulose;
c) from 0.1 to 5%, even more preferably 0.5%, 1%, 1.5%, 2%, 2.5%, 3%, 3.5%, 4%, 4.5% or 5%, of lubricants such as stearic acid or salt thereof, such as calcium stearate, zinc stearate, magnesium stearate, sodium stearyl fumarate, glyceryl monostearate, glyceryl palmitostearate and mixtures thereof;
d) from 0.1 to 5%, even more preferably 0.5%, 1%, 1.5%, 2%, 2.5%, 3%, 3.5%, 4%, 4.5% or 5%, of a glidant, such as microcrystalline wax, yellow beeswax, white beeswax and silicon dioxide;
e) from 0.1 to 5%, even more preferably 0.5%, 1%, 1.5%, 2%, 2.5%, 3%, 3.5%, 4%, 4.5% or 5%, of disintegrant, such as sodium starch glycolate, croscarmellose sodium, corn starch, crospovidone;
f) from 0.1 to 5%, even more preferably 0.5%, 1%, 1.5%, 2%, 2.5%, 3%, 3.5%, 4%, 4.5% or 5%, of surfactant, such as fatty acids and sodium lauryl sulfate;
g) from 1 to 20%, even more preferably 1%, 2%, 3%, 4%, 5%, 6%, 7%, 8%, 9%, 10%, 11%, 12%, 13%, 14%, 15%, 16%, 17%, 18%, 19%, 20% of a palatability enhancer selected from those known in the state of the art, such as natural or artificial palatability enhancers capable of stimulating one or more senses of domestic animals, such as smell, synesthesia, taste or texture, including, but not limited to palatability enhancers capable of simulating the taste of chicken, meat or vegetables and others, applicable to solid oral compositions, in particular the veterinary ones.

In one embodiment, the composition provides high bioavailability of poorly soluble active ingredients. In another embodiment, it provides compositions having a large quantity of the active ingredient greater than 30%, 40%, or even 50% in the composition. In another aspect, the composition of the present invention provides tablets of reduced size, from 5 to 50 mm, depending on the dosage of active ingredient used. In a different embodiment, said composition presents a unique ratio between the hardness of its solid composition and the amount of active ingredient between 0.5-1.5. In another embodiment, the composition is anhydrous.

Solid dosage forms such as tablets, dragées, capsules, pills, and granules can be prepared with coatings, such as enteric coatings and other coatings well known in the pharmaceutical formulation art. They may optionally contain opacifying agents and may further comprise a composition that releases the active ingredient(s) only or releases the active ingredient only in a specific part of the animal's intestinal tract, optionally in a prolonged manner.

### Treatment and prevention of diseases

The present invention comprises active ingredients that are poorly soluble in water and excipients, and may be obtained through a sustainable production process, being useful in the treatment and/or prevention of parasites, insects, and mites, preferably fleas and ticks, even more preferably ticks of the species *Rhipicephalus sanguineus sensu lato* and fleas of the species *Ctenocephalides felis felis*, as well as diseases potentially associated with these animals caused directly by them or through their infection by other pathogens.

Said composition may be used to prevent such diseases, depending on the dosage, the composition used, and the animal's condition. Thus, the composition of the present invention, when administered at the recommended dose, is effective in controlling fleas for at least 77 days and ticks for at least 63 days after treatment in domestic animals. Therefore, said composition can be used for monthly or bimonthly treatments of domestic animals.

### Process of manufacture

The present invention comprises a sustainable manufacture process for obtaining solid veterinary compositions comprising poorly soluble active ingredients.

Said process comprises solvent-free (including water-free) steps that do not require heating, comprising the following steps:
a) mixing part of the diluent with the active ingredient;
b) mixing part of the diluent with part of the glidant;
c) mixing a) with part of the diluent and homogenizing;
d) mixing b), the surfactant, disintegrant, binder, c) and homogenizing;
e) compacting the mixture of d);
f) obtaining granules and transferring the compacted mixture to the mixer of e);
g) adding and mixing with the remaining disintegrant, glidant, lubricant, total palatability enhancer, and diluent.

In one embodiment, the purpose of the process is to obtain a composition as described in the present invention.

### Examples

### Example 1

As previously stated, the manufacture process of the invention comprises a sustainable process that eliminates the use of solvents and heating, providing a product with unique pharmacotechnical and bioavailability features for active ingredients that are poorly water soluble. Ideally, the manufacture process comprises the following steps:
a) mixing part of the diluent with the active ingredient;
b) mixing part of the diluent with part of the glidant;
c) mixing a) with part of the diluent and homogenizing;
d) mixing b), the surfactant, disintegrant, binder, c) and homogenizing;
e) compacting the mixture of d);
f) obtaining granules and transferring the compacted mixture to the mixer of e);
g) adding and mixing with the remaining disintegrant, glidant, lubricant, total palatability enhancer, and diluent.

In particular, the manufacture process of the present invention comprises the following steps:
a) mixing 30 to 50% of the diluent with the active ingredient;
b) mixing 1 to 20% of the diluent with 40 to 60% of the glidant;
c) mixing a) with 40 to 60% of diluent and homogenizing;
d) mixing b), the surfactant, of 40 to 60% disintegrant, binder, c) and homogenizing;
e) compacting the mixture of d);
f) obtaining granules and transferring the compacted mixture to the mixer of e);
g) adding and mixing the remaining amount of disintegrant, glidant, lubricant, total palatability enhancer, and diluent.

In a preferred embodiment, the dry granulation process is carried out. In one aspect, the diluent(s), active ingredient(s), glidant(s), surfactant(s), disintegrant(s), binder(s) can be sieved individually or in combination, in whole or in part. In one embodiment, the diluent is sieved together with the active ingredient in step a). Preferably, the diluent is sieved together with the glidant in step b). Preferably, the diluent of step c) is previously sieved separately. In one embodiment, 35 to 45% of diluent is used in step a). In another embodiment, 5 to 15% of diluent is used in step b). In another embodiment, 1 to 10% of diluent is used in step g). In one embodiment, 50% of glidant is used in step b). In one embodiment, 50% of glidant is used in step g). Preferably, 45 to 55% of the diluent is used in step c). Preferably, 50% of disintegrant is used in step d). Preferably, 50% of disintegrant is used in step g). In a preferred embodiment, after step d), from 20 to 50% of the lubricant is added and homogenized before proceeding to step e). In a preferred embodiment, the remaining amount of lubricant is 80 to 50%. The active ingredient, surfactant, palatability enhancer, and binder are used in their entireties (100%) in the steps where they are required.

The manufacture process, in one embodiment, resulting in a product comprising the desired characteristics of bioavailability, hardness, size, average weight, and friability, as exemplified below:

| **Presentation** | **Hardness (kgf)** | **Friability (%)** | **Average weight (mg)** | **Size (diameter)** | **Hardness to size ratio** | **Active ingredient concentration to size ratio** |
|---|---|---|---|---|---|---|
| | | | | | | |
| 90 mg | 4-7 | up to 1.5 | 84.5-94.5 | 6 mm (round) | 1.08 | 7.5 |
| 200 mg | 5-10 | up to 1.5 | 190-210 | 8 mm (round) | 0.93 | 12.5 |
| 400 mg | 7-10 | up to 1.5 | 380-420 | 11.2 mm (round) | 0.75 | 17.85 |
| 800 mg | 8-12 | up to 1.5 | 760-840 | 13 mm (round) | 0.77 | 30 |
| 1120 mg | 8-14 | up to 1.5 | 1064-1176 | 19x9 mm (oblong) | 0.6 | 29.47 |

Wherein said composition, in another embodiment, may be obtained through means other than the aforementioned process.

Said solid oral composition of the present invention comprises the following elements and their respective concentrations:

| **Concentration (%)** | **Function** |
|---|---|
| 45 to 55% | Active ingredient |
| 1 to 10% | Binder |
| 0,1 to 5% | Glidant |
| 0,1 to 5% | Disintegrant |
| 0,1 to 5% | Surfactant |
| 1 to 20% | Palatability enhancer |
| 0,1 to 5% | Lubricant |
| q.s.p. | Diluent |

Examples of compositions comprising said elements may be seen below:

| **Composition 1** | | **Composition 2** | | **Composition 3** | | **Composition 4** | |
|---|---|---|---|---|---|---|---|
| **Compound** | **% wt/ wt** | **Compound** | **% wt/ wt** | **Compound** | **% wt/ wt** | **Compound** | **% wt/ wt** |
| Isoxazoline (Fluralaner) | 45-55 | Isoxazoline (Fluralaner) | 52 | Fluralaner | 50 | Isoxazoline (Fluralaner) | 49 |
| Cellulose derivatives | 1-10 | Hydroxypropylmethylc ellulose | 2-5 | Hydroxypropylcel lulose | 8 | Carboxymethylcel lulose | 1-6 |
| Silicon dioxide | 0.1-5 | Silicon dioxide | 0.1-5 | Silicon dioxide | 1 | Silicon dioxide | 0.1-5 |
| Sodium | 0.1- | Sodium Starch | 0.1- | Crospovidone | 3 | Sodium Starch | 0.1- |
| Starch Glycolate | 5 | Glycolate | 5 | | | Glycolate | 5 |
| Sodium lauryl sulfate | 0.1-5 | Sodium lauryl sulfate | 0.1-5 | Sodium lauryl sulfate | 1 | Sodium lauryl sulfate | 0.1-5 |
| Palatability enhancer | 1-20 | Palatability enhancer | 1-20 | Palatability enhancer | 10 | Palatability enhancer and | 1-20 |
| Stearic acid or salt thereof | 0.1-5 | Sodium stearyl fumarate | 0.5-2 | Sodium stearate | 1 | Zinc stearate | 0.5-2 |
| Dibasic calcium phosphate; Lactose | q.s. p. | Dibasic calcium phosphate | q.s. p. | Silicified Microcrystalline Cellulose | q.s. p. | Lactose | q.s. p. |

### Palatability enhancer

All study protocols were submitted to the Animal Ethics Committee of the respective Contract Research Organization (CRO). Each trial was initiated only upon approval. The trials took place at the CROs, whose facilities and management comply with the requirements of the chapter "Cães e gatos domésticos mantidos em instalações de instituições de ensino ou pesquisa cientifica" [*Domestic dogs and cats kept in facilities of educational or scientific research institutions*] of the Brazilian Guidelines for the Production, Maintenance or Use of Animals in Teaching or Scientific Research Activities from CONCEA (gov.br/mcti/pt-br/composicao/conselhos/concea/arquivos/arquivo/publicacoes-doconcea/guia_concea_1 ed_animais-_ensino_ou_pesquisa_2023.pdf).

The trials also included investigations of adverse events (any harmful, unintended response that may manifest itself as disease, abnormality, or injury, observed in animals participating in a clinical trial after administration of PVI, which may or may not be related to the use of PVI). No adverse events occurred during any of the trials. All animals included in the trials were assessed for their overall health, and were found to be healthy, without any abnormalities. Throughout the trials, all dogs were monitored daily for health, and no abnormalities were observed. For participation in the trial, only dogs weighing over 2.0 kg live weight, with an ideal body condition score, healthy, in good health and nutritional condition, were selected; females had to be neither pregnant nor lactating.

In this trial, 29 animals per group were used. Palatability of each product was tested on a panel of adult dogs, both male and female, of small, medium and large breeds. Each animal was given a tablet according to its weight in a single-day monadic test. Two criteria were assessed: prehension (immediate, taken by hand, or not taken) and consumption (partial, full, or not consumed). Acceptance is calculated based on the percentage of dogs that fully consume the product. Regarding the dogs' behavior, in all groups, the assistant commented that they all showed a strong attraction to their respective products and consumed them quickly. Prehension of the product of the present invention was 93.1% and acceptance of the product was 93.1%.

### Effectiveness

The trial was effectively formed by groups of six dogs each, following a randomized block design: animals were ranked in descending order of tick counts, forming blocks, within which they were designated to one of the groups by random selection, ensuring the formation of homogeneous groups. The experimental treatments were randomly assigned to the animals. The study was controlled (one of the groups was not medicated and was kept as a negative control), randomized in randomized blocks, and "blinded". Therefore, the researcher responsible for randomizing the dogs into groups and for administering the animals' treatments did not participate in the collection of post-treatment variables related to determining efficacy and investigating adverse events. Those responsible for all these post-treatment collections were blinded to the experimental groups and their respective treatments. The animals underwent a 21-day acclimation period to the study conditions, between the time of pre-selection and the start of treatment administration, during which they were identified and assessed for health, being observed at least once a day. All animals were clinically assessed at pre-selection to determine their overall health status, and were weighed on the same day, and again two days before treatment, to confirm the dose each individual should receive for their respective treatment. Along with each weighing, the body condition score of each animal was evaluated. Before weighing, the scale was checked against a known weight. The tablets were administered orally to pre-selected animals in their respective groups, and correct administration and swallowing were observed after administration, in a single dose. All animals received the appropriate dose for their weight range, specifically, oral tablets containing 100, 200, and 400 mg of the active ingredient (Fluralaner) were given to the animals in the efficacy and bioavailability trials. Flea and tick infestations in dogs occurred on days -7 and -2, pre-treatment, and 5, 12, 19, 26, 33, 40, 47, 54, 61, 68, 75, and 82 after treatment. At day -5, a count was performed, accompanied by the removal of parasites, to rank the animals and confirm their ability to retain fleas and ticks. After treatment, inspections/counts/removals were carried out 1 (at this time the parasites were not removed), 2, 7, 14, 21, 28, 35, 42, 49, 56, 63, 70, 77 and 84 days after treatment. The efficacy calculation was based on the number of live fleas and live attached ticks counted in the negative control and in each treated group, on each counting date. First, the count data were assessed for normality using the Shapiro-Wilk test. When the distribution was normal (parametric data - P > 0.05), the data was evaluated by comparing tick and flea counts between groups at all time points using Analysis of Variance (ANOVA). Non-parametric data (P < 0.05) were assessed using the Kruskal-Wallis test. The significance level considered in all tests was 95% (P < 0.05). For the animals in the group treated with the product of the present invention, no live fleas were found up to day 49 post-treatment, thus demonstrating 100% efficacy against fleas up to that point in the study. On days 56, 63, 70, and 77 post-treatment, the efficacy levels observed over the course of the days were 99.2%, 98.7%, 96.1%, and 90.2%, respectively. Therefore, the product of the present invention demonstrated 100% efficacy against fleas up to 49 days after treatment, and maintained efficacy greater than 90% up to 77 days after treatment. For this product, no live ticks were found attached to the animals up to 28 days post-treatment, demonstrating 100% efficacy against ticks during this period. On the remaining days post-treatment, efficacy of this presentation lasted until D+63, where the demonstrated efficacy was 95.4%. Therefore, regarding efficacy against ticks, the same product showed 100% efficacy up to 28 days after treatment, maintaining efficacy greater than 90% up to 63 days after treatment. Therefore, it can be concluded that the product, when administered at the recommended dose, is effective in controlling fleas for at least 77 days and ticks for at least 63 days after treatment.

### Bioavailability

A larger number of dogs were pre-selected to ensure the selection of animals suitable for participation in the study, using a randomized block design, forming two groups containing 12 dogs each, each group being homogeneous with respect to breed, category, gender, age and weight, ensuring that these factors did not interfere with the results. The animals underwent a seven-day acclimatization period between pre-selection and treatments. To form the groups, the selected animals were first divided into two blocks, according to gender, and listed in descending order of weight within each block. Animals of the same gender and weight were listed in ascending order of age. From this list, repetitions were formed within each block, and the animals within each repetition were assigned to one of the groups by random selection. The group to be treated with the product of the present invention was determined by random selection. These animals underwent daily general health checks, as well as clinical and laboratory examinations. Clinical examinations were carried out at two time points before treatment, 48 and 96 hours after treatment, and on days 7, 14, 28, 42, and 56. All animals were weighed at the same time points on a scale that has its calibration assessed and using a known standard weight. The animals were evaluated for possible signs of systemic intoxication both before treatment and 2, 4, 7, 14, 28, 42, and 56 days post-treatment. The weight obtained the day before treatment was used to calculate the dose for each animal, for the administration of treatments according to the proposed dosage. Treatments were administered concomitantly with feeding, as administration of the active ingredient to fed dogs increases the plasma bioavailability of the active ingredient. On day 0 (D0), the animals received wet feed (pâté) about 15 minutes before treatment, and the remaining half of the pâté immediately after administration of the product. To prevent the use of wet feed from impacting the study, the animals were acclimated to this type of diet. Blood samples were collected and sent for the analytical step, which involved quantifying the active ingredient in the plasma to calculate pharmacokinetic parameters and evaluate plasma bioavailability. Samples were collected from the dogs for quantification of the active ingredient at the following post-treatment time points: 0h (pre-administration - control sample); 2 hours; 4 hours; 8 hours; 1 day; 2 days; 3 days; 4 days; 7 days; 14 days; 21 days; 28 days; 35 days; 42 days; 49 days; 56 days; 63 days; 70 days; 77 days; 84 days; 91 days; 98 days; 105 days; and 112 days. At least 4 mL of blood were collected from each animal using disposable syringes and needles, and immediately after collection, the blood was placed in tubes containing an anticoagulant (sodium citrate). The samples were centrifuged immediately after collection and properly packaged for shipment to the contracted analytical laboratory to quantify the active ingredient.

The pharmacokinetic variables calculated after receiving the active ingredient concentrations in plasma, quantified by a duly validated method, were: ASC₀₋₂₄ₕ (area under the plasma concentration versus time curve, from time zero to 24 hours); ASC_{0-inf} (area under the plasma concentration versus time curve, from time zero to infinity); ASCₗₐₛₜ (area under the plasma concentration versus time curve, from time zero to the last sample collected); Cₘₐₓ (maximum concentration reached); Cₗₐₛₜ (last plasma concentration reached); Tₘₐₓ (time at which the maximum concentration was reached); T_{1/2} (elimination half-life); V_{d} (volume of distribution); Kₑₗ (elimination constant); CL ("clearance"). These were calculated using the PKSolver add-on package, developed in Visual Basic for Applications (VBA) and available for Microsoft Excel^{®} software.

An example of the results obtained can be seen in the table below:

| **Product** | **1^{st} Formulation (Active Ingredient Fluralaner)** | **2^{nd} Formulation (Active Ingredient Fluralaner)*** |
|---|---|---|
| **Average dose of Fluralaner** | **14.4 mg/kg** | **25 mg/kg** |
| Cₘₐₓ (ppb) | 4318 + 2289 | 3948 + 1734 |
| Tₘₐₓ (d)* | 1 (0.17-7) | (1-2) |
| AUC (0-t) | 49960 + 11614 | 46115 + 18932 |
| AUC (0-∞) | 51233 + 11999 | 46416 + 18929 |
| T1/2d | 11 +3 | 12 + 3 |

| | | |
|---|---|---|
| * data from the literature | | |

As seen in the table above, by comparing the bioavailability results of a formulation made according to the teachings of the present patent application with a second composition that does not have the same characteristics as the first composition, we find that the Cmax and AUC parameters of the composition with 10 to 25 mg/kg (average in this study of 14.4 mg/kg) of active ingredient show higher results than the Cmax and AUC of the composition with 25 mg/kg of active ingredient, indicating a greater bioavailability of the first composition, and this result may be caused by the distinct pharmaceutical form of the first composition, claimed herein.

A person skilled in the art will understand that the examples disclosed herein serve to corroborate the invention, but the invention is not necessarily limited to them.

## Claims

**1. A COMPOSITION** for domestic animals **characterized in that** it comprises:
i) 30 to 70% of at least one poorly water-soluble active ingredient;
ii) 30 to 70% of at least one excipient.

**2. The COMPOSITION,** according to claim 1, **characterized in that** the excipients comprise at least one of the following: binder, glidant, disintegrant, surfactant, palatability enhancer, lubricant and/or diluent.

**3. The COMPOSITION,** according to claim 1, **characterized in that** it comprises 45 to 55% of at least one poorly water soluble active ingredient.

**4. The COMPOSITION,** according to claim 2, **characterized in that** it comprises excipients comprising at least one of the following elements: 1 to 10% binder, 0.1 to 5% glidant, 0.1 to 5% disintegrant, 0.1 to 5% surfactant, 1 to 20% palatability enhancer, 0.1 to 5% lubricant and/or diluent q.s.p.

**5. The COMPOSITION,** according to claim 1 or 3, **characterized in that** it comprises a high concentration of poorly water soluble active ingredients selected from actives with water solubility lower than 1 mg/mL at 25°C, namely isoxazoline compounds such as fluralaner, sarolaner, afoxolaner, or lotilaner.

**6. The COMPOSITION,** according to claim 1, 2 or 4, **characterized in that** it comprises at least one element selected from the groups comprising: at least one pharmaceutically acceptable inert excipient or carrier or diluent, such as sodium citrate or dicalcium phosphate starches, lactose, sucrose, glucose, mannitol, silicic acid, talc and silicified microcrystalline cellulose; binders such as cellulose derivatives, carboxymethylcellulose, methylcellulose, hydroxypropylcellulose, hydroxypropylmethylcellulose; lubricants such as stearic acid or a salt thereof, calcium stearate, zinc stearate, magnesium stearate, sodium stearyl fumarate, glyceryl monostearate, glyceryl palmitostearate and mixtures thereof; glidants such as microcrystalline wax, yellow beeswax, white beeswax and silicon dioxide; disintegrants such as sodium starch glycolate, croscarmellose sodium, corn starch, crospovidone; surfactants, such as fatty acids and sodium lauryl sulfate.

**7. The COMPOSITION,** according to claim 1, **characterized in that** it provides a composition with high bioavailability of poorly soluble active ingredients.

**8. The COMPOSITION,** according to claim 1, **characterized in that** it provides tablets of reduced size of from 5 to 50 mm, depending on the dosage of active ingredient used.

**9. The COMPOSITION,** according to claim 1, **characterized in that** it has a diameter of less than 10 mm for compositions up to 200 mg, less than 15 mm for compositions from 200 to 800 mg, and less than 30 mm for compositions from 800 mg to 1200 mg.

**10. The COMPOSITION,** according to claim 1, **characterized in that** it presents a hardness to amount of active ingredient ratio in the composition between 0.5-1.5.
**The COMPOSITION,** according to claim 1, **characterized in that** it is an anhydrous solid composition.

**12. A PROCESS for the manufacture** of the composition of claim 1 **characterized by** comprising the steps of:
a) mixing part of the diluent with the active ingredient;
b) mixing part of the diluent with part of the glidant;
c) mixing a) with part of the diluent and homogenizing;
d) mixing b), the surfactant, disintegrant, binder, c) and homogenizing;
e) compacting the mixture of d);
f) obtaining granules and transferring the compacted mixture to the mixer of e);
g) adding and mixing with the remaining disintegrant, glidant, lubricant, total palatability enhancer, and diluent.

**13. The PROCESS,** according to claim 12, **characterized in that** it is dry granulation.

**14. The PROCESS,** according to claim 12, **characterized in that** it is a sustainable process that does not require the use of heating and solvents.

**15. The PROCESS** according to claim 12 **characterized by** comprising the steps of:
a) mixing 30 to 50% of the diluent with the active ingredient;
b) mixing 1 to 20% of the diluent with 40 to 60% of the glidant;
c) mixing a) with 40 to 60% of diluent and homogenizing;
d) mixing b), the surfactant, of 40 to 60% disintegrant, binder, c) and homogenizing;
e) compacting the mixture of d);
f) obtaining granules and transferring the compacted mixture to the mixer of e);
g) adding and mixing with the remaining disintegrant, glidant, lubricant, total palatability enhancer, and diluent.

**16. USE** of a composition of claim 1 **characterized in that** it is for the preparation of a medicament for the treatment and prevention of infestations by parasites, insects and/or mites, as well as diseases potentially related to these animals, caused directly by them or through their infection by other pathogens.

**17. USE,** according to claim 16, **characterized in that** it is for the treatment or prevention of flea and/or tick infestations.

**18. USE,** according to claim 16, **characterized in that** it is for the treatment or prevention of infestation caused by ticks of the species *Rhipicephalus sanguineus* and fleas of the species *Ctenocephalides felis.*

**19. USE,** according to claim 17, **characterized in that** it is effective for the treatment or prevention of infestation for at least 77 days and against ticks for at least 63 days.
